(19) Europäisches Patentamt
European Patent Office
Office européen des brevets

(11) **EP 3 042 193 B1**

(12) **EUROPÄISCHE PATENTSCHRIFT**

(45) Veröffentlichungstag und Bekanntmachung des Hinweises auf die Patenterteilung:
**01.05.2019 Patentblatt 2019/18**

(21) Anmeldenummer: **14772274.8**

(22) Anmeldetag: **25.08.2014**

(51) Int Cl.:
*G01N 33/00* [(2006.01)]    *G01F 1/36* [(2006.01)]
*G01F 1/68* [(2006.01)]    *G01N 7/00* [(2006.01)]
*G01N 11/08* [(2006.01)]

(86) Internationale Anmeldenummer:
**PCT/DE2014/000425**

(87) Internationale Veröffentlichungsnummer:
**WO 2015/032377 (12.03.2015 Gazette 2015/10)**

(54) **VORRICHTUNG UND VERFAHREN ZUR BESTIMMUNG VON MISCHUNGSVERHÄLTNISSEN STRÖMENDER GASMISCHUNGEN**

DEVICE AND METHOD FOR DETERMINING MIXING RATIOS OF FLOWING GAS MIXTURES

DISPOSITIF ET PROCÉDÉ DE DÉTERMINATION DE PROPORTIONS D'UN MÉLANGE DE GAZ EN ÉCOULEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.09.2013 DE 102013014532**

(43) Veröffentlichungstag der Anmeldung:
**13.07.2016 Patentblatt 2016/28**

(73) Patentinhaber: **W.O.M. World of Medicine GmbH 10587 Berlin (DE)**

(72) Erfinder:
• **KÖLM, Matthias**
**10315 Berlin (DE)**

• **JÜLG, Peter**
**79098 Freiburg (DE)**

(74) Vertreter: **Jungblut & Seuss Patentanwälte Max-Dohrn-Strasse 10 10589 Berlin (DE)**

(56) Entgegenhaltungen:
EP-A1- 1 686 355        EP-A2- 0 022 493
WO-A2-00/14484          JP-A- 2008 116 283
US-A- 2 439 723         US-A- 4 576 043
US-A1- 2009 293 634

**Beschreibung**

Die vorliegende Erfindung betrifft eine Vorrichtung und ein Verfahren zur Bestimmung des $CO_2$-Anteils von strömender Luft in medizintechnischen Vorrichtungen durch Verwendung zweier Strömungswiderstände mit verschiedenen Kennlinien.

Die Bestimmung der Anteile verschiedener Komponenten von Fluiden, beispielsweise Gas- oder Flüssigkeitsmischungen ist auf vielen Gebieten der Technik nötig. Hierzu gibt es eine breite Vielfalt von verschiedenen Methoden, unter Anwendung optischer, akustischer oder elektrochemischer Verfahren. Gleichwohl fehlt es an einem einfachen Verfahren zur Bestimmung der Mischungsverhältnisse zweier Fluide, insbesondere zweier Gase. Gegenstand der vorliegenden Erfindung ist daher ein einfaches Verfahren zur Bestimmung des $CO_2$-Anteils von strömender Luft in medizintechnischen Vorrichtungen und eine hierzu angepasste Vorrichtung.

Die vorliegende Erfindung lehrt daher eine Vorrichtung gemäß Anspruch 1 sowie ein mittels dieser Vorrichtung durchgeführtes Verfahren gemäß Anspruch 5. Vorteilhafte Ausgestaltungen der Erfindung sind Gegenstand der darauf bezogenen Unteransprüche.

Die nachfolgend beispielhaft beschriebene Vorrichtung und das mittels der Vorrichtung durchgeführte Verfahren ist in der Lage die relativen Bestandteile einer Gasmischung präzise anzugeben, z. B. die Anteile einer Gasmischung von $CO_2$ und Stickstoff. Die Erfindung beruht auf der Tatsache, dass es Strömungswiderstände mit unterschiedlichen Kennlinien gibt. Beispielhaft genannt seien hier ein Sintermetallfilter und eine Lochblende. Der Strömungswiderstand eines Sintermetallfilters weist eine annähernd lineare Kennlinie auf, d. h. der Strömungswiderstand steigt annähernd linear mit der Strömungsgeschwindigkeit. Im Gegensatz dazu weist eine Lochblende eine annähernd quadratische Kennlinie auf, d. h. der Strömungswiderstand wächst annähernd mit der Strömungsgeschwindigkeit zum Quadrat. Diese unterschiedlichen Kennlinien sind vermutlich auf unterschiedliche Strömungsverhältnisse bei der Filterpassage zurückzuführen: Es ist anzunehmen, dass innerhalb des Sintermetallfilters eine annähernd laminare Strömung herrscht, so dass der Strömungswiderstand im Wesentlichen durch die Gasviskosität bestimmt ist. Im Gegensatz dazu dürfte die Strömung an einer Lochblende turbulent sein, so dass der Strömungswiderstand vor allem durch die Gasdichte bestimmt wird. Hierbei handelt es sich allerdings lediglich um theoretische Überlegungen ohne Anspruch auf Richtigkeit. In jedem Fall kann die oben beispielhaft genannte Lochblende auch durch andere Strömungswiderstände ersetzt werden, beispielsweise durch Verengungen, Ventile, Biegungen, Wedge-Elemente oder Venturi-Rohre. Das Sintermetallfilter kann auch durch andere Vorrichtungen ersetzt werden, welche eine annähernd laminare Strömung gewährleisten.

Als Alternative zur Messung des Differenzdruckes kann auch die Messung des Massedurchflusses, beispielsweise mittels eines thermischen Gasflusssensors, erfolgen.

Erfindungsgemäß ist es möglich komplexere Gasmischungen zu analysieren, beispielsweise Mischungen aus "Luft" und $CO_2$. Hierbei wird allerdings vorausgesetzt, dass die Mischungsverhältnisse von Luft (bzw. einem anderen Gasgemisch) konstant sind. Am Beispiel des Mischungsverhältnisses von "Luft" und $CO_2$ wird außerdem der "natürliche" $CO_2$-Anteil der Luft (ca. 0,04%) vernachlässigt.

Theoretischer Ansatz

Aufgrund der Abhängigkeit des Strömungswiderstandes von den Strömungseigenschaften des Mediums, kann die Zusammensetzung von Gasgemischen ermittelt werden. Voraussetzung ist, dass die Strömungseigenschaften der Gase sich signifikant voneinander unterscheiden. Grundsätzlich ist dieser Zusammenhang auch auf Flüssigkeiten anwendbar.

Das Dokument US 4,576,043 offenbart eine Bestimmung des Phasenverhältnisses eines Zweiphasenstroms in der Erdöltechnik mittels des Druckabfalls an zwei unterschiedlichen Strömungswiderständen. Das Dokument JP 2008-116283A offenbart eine ähnliche Bestimmung des Strömungsflusses und des Volumenverhältnisses von Brenngas/Luft-Gemischen in Verbrennungsmaschinen mittels des Druckabfalls an zwei unterschiedlichen Strömungswiderständen.

Mess- und Kalibrieraufbau

Zur Ermittlung der Konzentrationsverhältnisse, wird das Medium durch eine Reihenschaltung von zwei Strömungswiderständen geleitet. Dabei wird der jeweils über dem Strömungswiderstand abfallende Differenzdruck registriert und als Grundlage zur Ermittlung der Konzentrationsverhältnisse genutzt. Voraussetzung für die Unterscheidung verschiedener Medienanteile, ist das Verwenden zweier Strömungswiderstände mit unterschiedlicher Kennlinie, z.B. linear und quadratisch. In dem hier dargestellten Messaufbau wird dies durch einen Sintermetallfilter (linear) und eine Blende (quadratisch) realisiert. Darüber hinaus wird über einen Drucksensor der Absolutdruck (p_abs) aufgenommen, mit welchem durch Druckschwankungen entstehende Fehler kompensiert werden (siehe unten). Ein weiterer Bestandteil des Aufbaus ist eine Temperaturmessung des Gases, zur Korrektur eventueller Temperaturschwankungen (siehe unten).

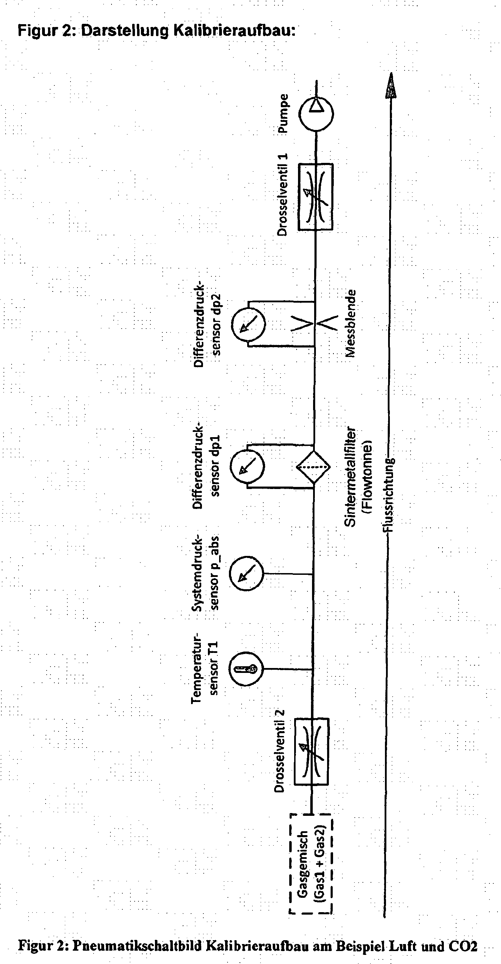

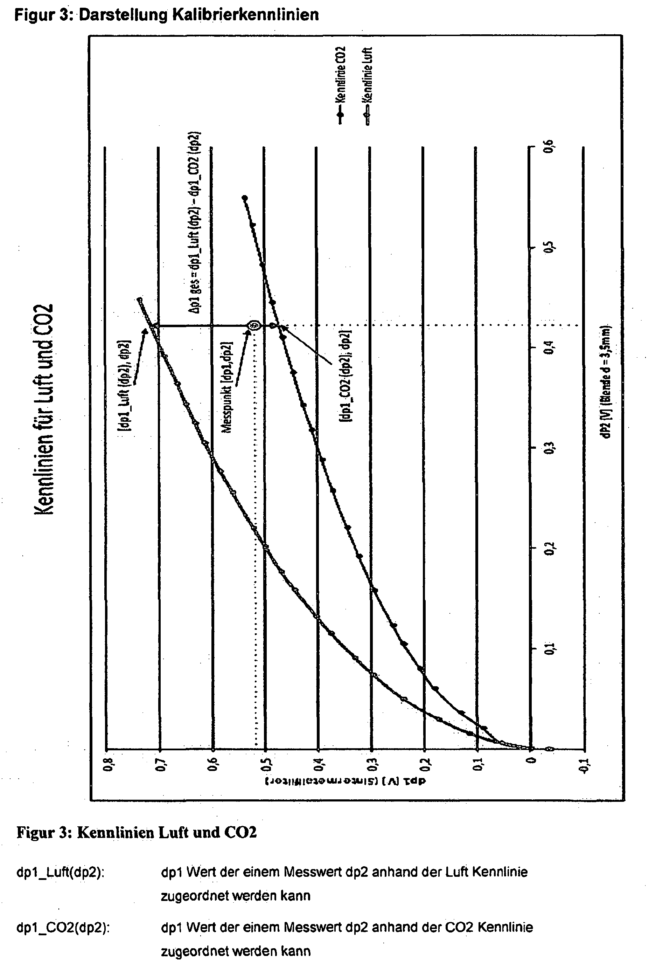

Zur Nutzung des Messaufbaus ist ein vorheriges Kalibrieren mit den entsprechenden Medien erforderlich. Dies ist jeweils mit den beiden zu messenden Mischungskomponenten notwendig. Ein beispielhafter Aufbau zur Kalibrierung wird in der Figur 2 gezeigt. Durch das Auftragen des Differenzdruckes dp1 über den Differenzdruck dp2 entsteht pro Medium eine Kennlinie (Figur 3), welche für die spätere Berechnung der Konzentrationsanteile genutzt wird (siehe unten).

Zur Erzeugung eines Volumenstromes wird eine Pneumatikpumpe genutzt, wobei die Steuerung des Volumenstromes über Drosselventil 1 erfolgt. Das Drosselventil 2 dient der Erzeugung verschiedener Unterdruckniveaus über die gesamte Messstrecke, zur Ermittlung der Parameter der Absolutdruckkorrektur (siehe unten). Am Beispiel des hier zu messenden Gas-Gemisches aus Gas 1 und Gas 2 erfolgt eine Kalibrierung mit reinem Gas 1 bzw. reinem Gas 2. Die aus einer derartigen Mischung ermittelten Kennlinien sind in Figur 3 am Beispiel einer Mischung aus Luft (Gas 1) und $CO_2$ (Gas 2) dargestellt.

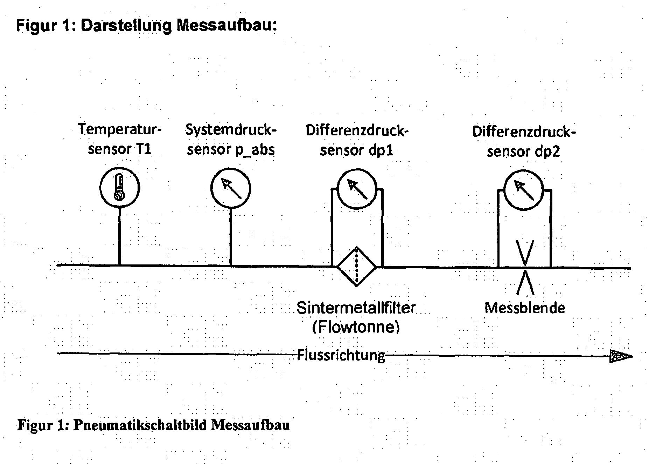

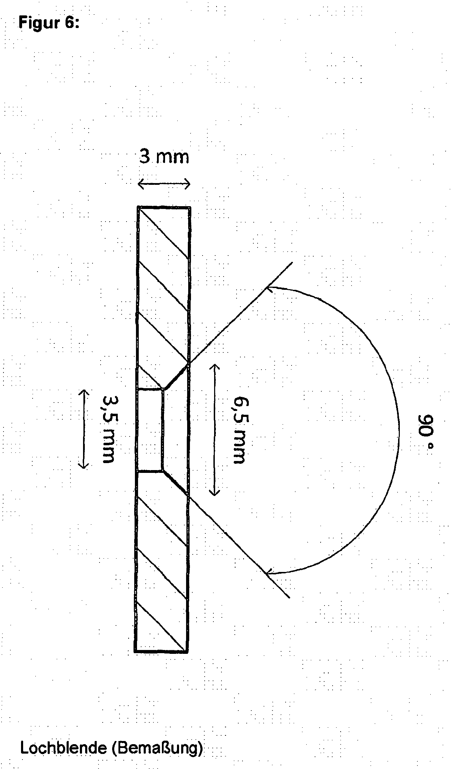

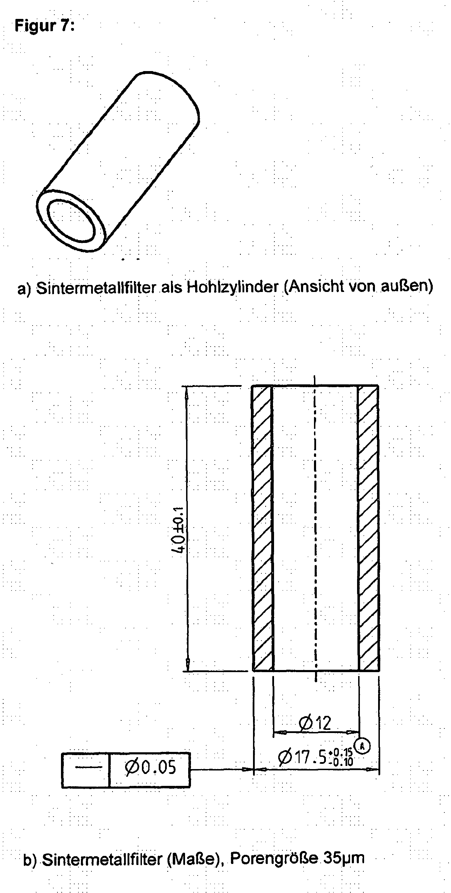

Hierbei wurde beispielhaft eine Kalibrierung und nachfolgende Messung eines Luft/CO2 Gemisches vorgenommen. Die Gasleitungen hatten einen Durchmesser von 8-10 mm. Es wurden Gasflüsse bis zu 30l/min realisiert. Vor der Messstrecke wurden (siehe Figur 1) wurden Drücke von bis zu -150mbar relativ zur Atmosphäre gemessen, mittels eines Sensors aus der HDI Reihe der Firma Sensotronics, welcher im Bereich von -1 bar bis + 1 bar arbeitet. Die Differenzdrucksensoren über den Strömungswiderständen hatten einen Messbereich von 0-10 mbar. Hierzu wurden Sensoren der HDI Reihe der Firma Sensotronics Benutzt (HDIM010DUF8P5). Als Strömungswiderstände wurde eine Lochblende (Figur 6) sowie ein Sintermetallfilter (Figur 7) mit einer Porengröße von 35$\mu$m verwendet. Wie in Figur 7 ersichtlich war der Sintermetallfilter in Form eines Hohlzylinders bestehend aus Cr-Ni-Stahl ausgestaltet.

Es wurde gefunden, dass eine für viele Zwecke ausreichende Genauigkeit der Kalibrierung bereits durch Kalibrierung mit nur einem Gas erreicht werden kann, wobei die Kennlinie des zweiten Gases errechnet werden kann. Dies ermöglicht beispielsweise eine regelmäßige Kalibrierung für eine Luft/$CO_2$-Mesung durch Bestimmung der Kennlinie für Luft unter Berechnung der Kennlinie für $CO_2$. Auf diese Weise kann die ansonsten nötige Bestimmung der $CO_2$-Kennlinie entfallen.

Grundgleichungen

Es gelten die folgenden Gleichungen bzw. Bedingungen:

i.) $$R = \frac{dP}{Q} ;$$ gilt für kleine Druckänderungen dp bzw. für lineare Strömungswiderstände R

ii.) $$\frac{c[Gas\,1]}{c[CO_2]} = \frac{R_{Gas\,1}}{R_{Gas\,2}} ;$$ c = Konzentration, R = Strömungswiderstand

iii.) $$\frac{dp2_{Gas\,2}}{R2_{Gas\,2}} = \frac{dp1_{Gas\,2}}{R1_{Gas\,2}} \neq \frac{dp2_{Gas\,1}}{R2_{Gas\,1}} = \frac{dp1_{Gas\,1}}{R1_{Gas\,1}}$$

In Worten: Die Kennlinien der Strömungswiderstände unterscheiden sich signifikant in Abhängigkeit der Medienkonzentration.

Berechnung des Mischungsverhältnis an einem Beispiel Gas 1 und Gas 2

In den folgenden Berechnungen stellt der Gas 2-Anteil die Zielgröße dar, welche ermittelt werden soll. Liegt eine Mischung aus Gas 1 und Gas 2 vor, setzten sich die Strömungswiderstände näherungsweise additiv aus den Strömungswiderständen $R_{Gas\,2}$ und $R_{Gas\,1}$ zusammen:

$$R1_{misch} = n * R1_{Gas\,1} + (1 - n) * R1_{Gas\,2}$$

$$R2_{misch} = n * R2_{Gas\,1} + (1 - n) * R2_{Gas\,2}$$

n := Anteil von Gas 1 bzw. Gas 2, $0 \leq n \leq 1$

Für die Messung eines Gasgemisches folgt aus (i.):

$$\frac{dp2}{R2_{misch}} = \frac{dp1}{R1_{misch}}$$

$$\frac{dp2}{dp1} = \frac{R2_{misch}}{R1_{misch}}$$

$$\frac{dp2}{dp1} = \frac{n * R2_{Gas\,1} + (1-n) * R2_{Gas\,2}}{n * R1_{Gas\,1} + (1-n) * R1_{Gas\,2}}$$

$$\frac{dp2}{dp1} = \frac{n * R2_{Gas\,1} + R2_{Gas\,2} - n * R2_{Gas\,2}}{n * R1_{Gas\,1} + R1_{Gas\,2} - n * R1_{Gas\,2}}$$

Mit $R = \frac{dp}{Q}$ folgt:

$$\frac{dp2}{dp1} = \frac{(n * dp2_{Gas\,1} + dp2_{Gas\,2} - n * dp2_{Gas\,2}) * Q}{(n * dp1_{Gas\,1} + dp1_{Gas\,2} - n * dp1_{Gas\,2}) * Q}$$

$$\frac{dp2}{dp1} = \frac{n * dp2_{Gas\,1} + dp2_{Gas\,2} - n * dp2_{Gas\,2}}{n * dp1_{Gas\,1} + dp1_{Gas\,2} - n * dp1_{Gas\,2}}$$

Annahme vertikaler Konzentrationsverlauf:

Für die Berechnung der Gaskonzentration wird die folgende Annahme getroffen:

$$dp2 = dp2_{Gas\,2} = dp2_{Gas\,1}$$

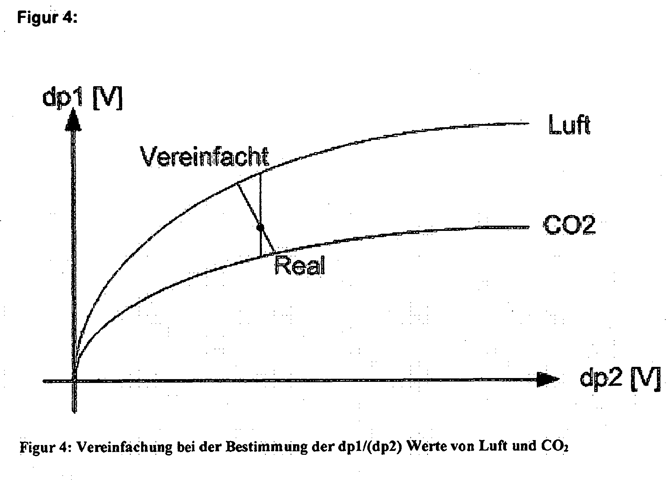

Dies entspricht einer Vereinfachung, da der reale Konzentrationsverlauf von einer zur anderen Kennlinie, also z.B. von 100% Gas 2 zu 100% Gas 1 nicht vertikal verläuft, sondern entlang einer Schrägen. Dieser experimentell entdeckte Effekt wird in Figur 4 dargestellt:
Diese Vereinfachung zur Ermittlung der Gas 2-Konzentration ist zulässig, da die Kennlinien von Gas 1 und Gas 2 in einem hinreichend kleinen Intervall als parallel zueinander angenommen werden können. Dadurch lässt sich der erste Strahlensatz der Elementargeometrie anwenden:

*Bei zwei durch einen gemeinsamen Punkt verlaufenden Geraden, welche von zwei Parallelen geschnitten werden, welche nicht durch den Schnittpunkt der Geraden verlaufen, gilt:*
*Die zwei durch den Geradenschnittpunkt und die Parallelen erzeugten Abschnitte einer Geraden verhalten sich so zueinander, wie die entsprechenden Abschnitte auf der zweiten Gerade.*

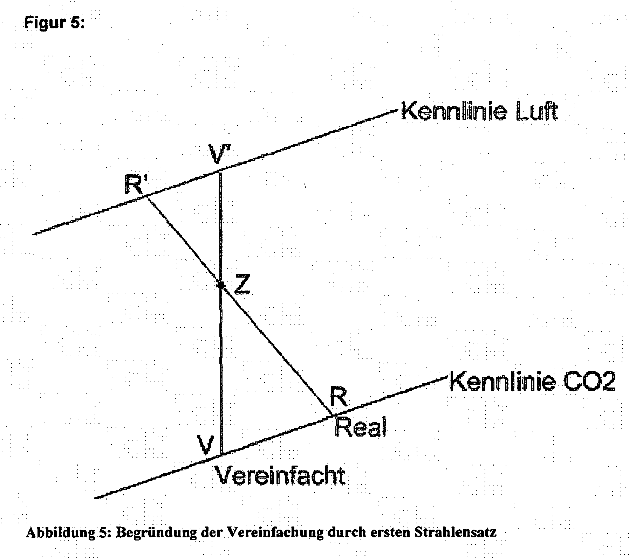

Angewandt auf den hier besprochenen Fall ergibt sich damit aus Figur 5 folgender Zusammenhang (auch hier beispielhaft dargestellt anhand der Kennlinien von Luft und $CO_2$):

$$\overline{VZ} : \overline{VV'} = \overline{RZ} : \overline{RR'}$$

Damit folgt:

$$\frac{dp2}{dp1} = \frac{n * dp2 + dp2 - n * dp2}{n * dp1_{Gas\,1} + dp1_{Gas\,2} - n * dp1_{Gas\,2}}$$

$$\frac{dp2}{dp1} = \frac{dp2}{n * dp1_{Gas\,1} + dp1_{Gas\,2} - n * dp1_{Gas\,2}}$$

$$\frac{dp2}{dp1} = \frac{dp2}{n * (dp1_{Gas\,1} - dp1_{Gas\,2}) + dp1_{Gas\,2}}$$

$$dp1 = n * (dp1_{Gas\,1} - dp1_{Gas\,2}) + dp1_{Gas\,2}$$

$$n = \frac{dp1 - dp1_{Gas\,2}}{(dp1_{Gas\,1} - dp1_{Gas\,2})}$$

$$n = \frac{dp1 - dp1_{Gas\,2}}{\Delta P1_{ges}}$$

Grenzwertbetrachtung:

$$dp1 = dp1_{Gas\,2} \Rightarrow n = 0 \Rightarrow Gas\,2 - Gehalt\ 100\%$$

$$dp1 = dp1_{Gas\,1} \Rightarrow n = 1 \Rightarrow Gas\,2 - Gehalt\ 0\%$$

Normierung:

Um die Gas 2 - Konzentration direkt anzugeben, wird wie folgt umgeformt:

$$Gas\ 2 - Konzentration = (1 - n) * 100\%[Gas\ 2]$$

$$= 1 - \frac{dp1 - dp1_{Gas\ 2}}{(dp1_{Gas\ 1} - dp1_{Gas\ 2})} * 100\%[Gas\ 2]$$

$$= \frac{\Delta p1_{ges}}{\Delta p1_{ges}} - \frac{dp1 - dp1_{Gas\ 2}}{\Delta p1_{ges}} * 100\%[Gas\ 2]$$

$$= \frac{(dp1_{Gas\ 1} - dp1_{Gas\ 2})}{\Delta p1_{ges}} - \frac{(dp1 - dp1_{Gas\ 2})}{\Delta p1_{ges}} * 100\%[Gas\ 2]$$

$$= \frac{(dp1_{Gas\ 1} - dp1_{Gas\ 2})}{\Delta p1_{ges}} + \frac{-dp1 + dp1_{Gas\ 2_2}}{\Delta p1_{ges}} * 100\%[Gas\ 2]$$

$$= \frac{(dp1_{Gas\ 1} - dp1)}{\Delta p1_{ges}} * 100\%[Gas\ 2]$$

$dp1_{Gas\ 1}$ *und* $dp1_{Gas\ 2}$ *müssen mit dem jeweiligen Messwert dp2 aus den Kalibrierkennlinien ermittelt werden!* $dp1$ *ist ein Messwert.*

Absolutdruckkorrektur

Absolutdruckschwankungen führen zur Zustandsänderung des Gas-Gemisches. Damit geht eine Änderung der Strömungseigenschaften einher, wodurch ein Messfehler hervorgerufen wird. Dieser Fehler ist entsprechend zu korrigieren. Hierfür wird mit dem dargestellten Kalibrieraufbau experimentell eine Korrekturformel bestimmt.

Temperaturkorrektur

Analog zu Absolutdruckschwankungen wirkt sich eine Temperaturschwankung auf das Messergebnis aus. Die Korrektur erfolgt mittels Nutzung der Temperatur in einer experimentell bestimmten Korrekturformel.

Verfahren

Das erfindungsgemäße Messverfahren basiert daher auf der Messung des Druckabfalls einer Gasströmung an zwei Strömungswiderständen im Vergleich mit einer Kalibrierkurve. Zur Durchführung des Verfahrens muss die aufgebaute Vorrichtung daher zunächst mittels zweier Gase kalibriert werden. Die genannten Gase können ihrerseits Mischungen darstellen, vorausgesetzt, dass das Mischungsverhältnis konstant bleibt, wie beispielsweise bei der Verwendung von Luft.
Das erfindungsgemäße Verfahren erlaubt die Bestimmung des $CO_2$-Anteils von Luft in medizintechnischen Vorrichtungen, z. B. Beatmungsgeräten oder Insufflatoren.

**EP 3 042 193 B1**

**Patentansprüche**

1. Vorrichtung zur Bestimmung des $CO_2$-Anteils von strömender Luft in medizintechnischen Vorrichtungen, **gekennzeichnet durch**, eine Strömungsleitung welche mindestens zwei Strömungswiderstände mit jeweils einem Differenzdrucksensor enthält welche in Reihe geschaltet sind, wobei die mindestens zwei Strömungswiderstände unterschiedliche Kennlinien aufweisen.

2. Vorrichtung gemäß Anspruch 1, wobei einer der Strömungswiderstände eine lineare Kennlinie und ein anderer Strömungswiderstand eine quadratische Kennlinie aufweist.

3. Vorrichtung gemäß Anspruch 1 oder 2, wobei ein Strömungswiderstand durch ein Sintermetallfilter und ein anderer Strömungswiderstand durch eine Lochblende gebildet wird.

4. Vorrichtung gemäß mindestens einem der Ansprüche 1-3, als Bestandteil eines medizintechnischen Insufflators oder eines Beatmungsgerätes.

5. Verfahren zur Bestimmung des $CO_2$-Anteils von strömender Luft in medizintechnischen Vorrichtungen, **dadurch gekennzeichnet, dass** die Luft durch eine Strömungsleitung geleitet wird, welche mindestens zwei Strömungswiderstände enthält, wobei mindestens ein Strömungswiderstand einen Differenzdrucksensor enthält und der andere Strömungswiderstand entweder einen Differenzdrucksensor oder einen Massenflusssensor enthält, welche in Reihe geschaltet sind, wobei die mindestens zwei Strömungswiderstände unterschiedliche Kennlinien aufweisen und wobei die Gaskonzentration durch Bestimmung des jeweiligen Druckabfalls an den Strömungswiderständen mittels einer Kalibrierkurve bestimmt wird.

6. Verfahren gemäß Anspruch 5, wobei einer der Strömungswiderstände eine lineare Kennlinie und ein anderer Strömungswiderstand eine quadratische Kennlinie aufweist.

7. Verfahren gemäß Anspruch 5 oder 6, wobei ein Strömungswiderstand durch ein Sintermetallfilter und ein anderer Strömungswiderstand durch eine Lochblende gebildet wird.

8. Verfahren gemäß Anspruch 5, wobei einer der Strömungswiderstände einen thermischen Masseflussensor aufweist.

9. Verfahren gemäß mindestens einem der Ansprüche 5-8, bei der medizintechnischen Beatmung oder Insufflation.


**Claims**

1. A device for determining the $CO_2$ content of flowing air in medical devices, **characterized by** a flow line containing at least two flow resistors having one differential pressure sensor each connected in series, the at least two flow resistors having different characteristic curves.

2. The device of claim 1, wherein one of the flow resistors has a linear characteristic curve, and another flow resistor has a square characteristic curve.

3. The device of claim 1 or 2, wherein one flow resistor is formed by a sinter-metal filter, and another flow resistor is formed by a pinhole aperture.

4. The device of at least one of claims 1 to 3, as a part of a medical insufflator or of a breathing apparatus.

5. A method for determining the $CO_2$ content of flowing air in medical devices, **characterized by** that the air is directed through a flow line containing at least two flow resistors, at least one flow resistor including a differential pressure sensor and the other flow resistor including either a differential pressure sensor or a mass flow sensor connected in series, wherein the at least two flow resistors have different characteristic curves, and wherein the gas concentration is determined by determining the respective pressure drop at the flow resistors by means of a calibration curve.

6. The method of claim 5, wherein one of the flow resistors has a linear characteristic curve, and another flow resistor has a square characteristic curve.

**7.** The method of claim 5 or 6, wherein one flow resistor is formed by a sinter-metal filter, and another flow resistor is formed by pinhole aperture.

**8.** The method of claim 5, wherein one of the flow resistors comprises a thermal mass flow sensor.

**9.** The method of at least one of claims 5 to 8, for medical breathing or insufflation.

**Revendications**

**1.** Dispositif de détermination de la teneur en $CO_2$ d'air circulant dans des dispositifs médicaux, caractérisé en une conduite de circulation contenant au moins deux résistances à l'écoulement comportant un capteur de pression différentielle chacun raccordés en série, les au moins deux résistances à l'écoulement ayant des courbes caractéristiques différentes.

**2.** Dispositif selon la revendication 1, dans lequel une des résistances à l'écoulement a une courbe caractéristique linéaire, et une autre résistance à l'écoulement a une courbe caractéristique carrée.

**3.** Dispositif selon la revendication 1 or 2, dans lequel une résistance à l'écoulement est formée par un filtre de métal fritté, et une autre résistance à l'écoulement est formée par un diaphragme perforé.

**4.** Dispositif selon au moins une des revendications 1 à 3, comme une partie d'un insufflateur ou d'un dispositif de respiration médical.

**5.** Procédé de détermination de la teneur en $CO_2$ d'air circulant dans des dispositifs médicaux, **caractérisé en ce que** l'air est guidée à travers une conduite de circulation contenant au moins deux résistances à l'écoulement, au moins une résistance à l'écoulement comportant un capteur de pression différentielle et l'autre résistance à l'écoulement comportant un capteur de pression différentielle ou bien un capteur de débit en masse raccordés en série, dans lequel les au moins deux résistances à l'écoulement ont des courbes caractéristiques différentes, et dans lequel la concentration en gaz est déterminée par la détermination de la chute de pression respective aux résistances à l'écoulement au moyen d'une courbe de calibration.

**6.** Procédé selon la revendication 5, dans lequel une des résistances à l'écoulement a une courbe caractéristique linéaire, et une autre résistance à l'écoulement a une courbe caractéristique carrée.

**7.** Procédé selon la revendication 5 ou 6, dans lequel une résistance à l'écoulement est formée par un filtre de métal fritté, et une autre résistance à l'écoulement est formée par un diaphragme perforé.

**8.** Procédé selon la revendication 5, dans lequel une des résistances à l'écoulement comprend un capteur de débit en masse thermique.

**9.** Procédé selon au moins une des revendications 5 à 8, pour la respiration ou insufflation médicale.

## Figur 1: Darstellung Messaufbau:

Figur 1: Pneumatikschaltbild Messaufbau

**Figur 2: Darstellung Kalibrieraufbau:**

Figur 2: Pneumatikschaltbild Kalibrieraufbau am Beispiel Luft und CO2

EP 3 042 193 B1

## Figur 3: Darstellung Kalibrierkennlinien

**Figur 3: Kennlinien Luft und CO2**

dp1_Luft(dp2):  dp1 Wert der einem Messwert dp2 anhand der Luft Kennlinie zugeordnet werden kann

dp1_CO2(dp2):  dp1 Wert der einem Messwert dp2 anhand der CO2 Kennlinie zugeordnet werden kann

**Figur 4:**

Figur 4: Vereinfachung bei der Bestimmung der dp1/(dp2) Werte von Luft und $CO_2$

Figur 5:

Abbildung 5: Begründung der Vereinfachung durch ersten Strahlensatz

**Figur 6:**

Lochblende (Bemaßung)

**Figur 7:**

a) Sintermetallfilter als Hohlzylinder (Ansicht von außen)

b) Sintermetallfilter (Maße), Porengröße 35µm

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4576043 A
- JP 2008116283 A